(19) Europäisches Patentamt
European Patent Office
Office européen des brevets

(11) Veröffentlichungsnummer: **0 122 412**
**A1**

(12) # EUROPÄISCHE PATENTANMELDUNG

(21) Anmeldenummer: 84102010.0

(22) Anmeldetag: 27.02.84

(51) Int. Cl.³: C 08 G 65/44
C 07 C 43/295, C 07 C 41/01

(30) Priorität: 09.03.83 DE 3308421

(43) Veröffentlichungstag der Anmeldung: 24.10.84 Patentblatt 84/43

(84) Benannte Vertragsstaaten: DE FR GB IT

(71) Anmelder: BAYER AG
Konzernverwaltung RP Patentabteilung
D-5090 Leverkusen 1 Bayerwerk(DE)

(72) Erfinder: Heitz, Walter, Prof. Dr.
Am Schmidtborn 5
D-3575 Kirchhain 1(DE)

(72) Erfinder: Risse, Wilhelm
Weidenhäuserstrasse 72
D-3550 Marburg(DE)

(54) **Verfahren zur Herstellung von bifunktionellen Polyphenylenethern.**

(57) Verfahren zur Herstellung von bifunktionellen Polyphenylenoxiden der Formel (I)

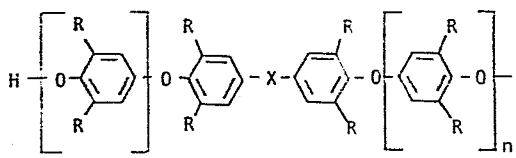

in welcher (I),

R gleich oder verschieden ist und für Wasserstoff, einen Alkylrest mit 1 bis 4 C-Atomen, oder einen Arylrest mit 6 C-Atomen steht,

X für eine Gruppe R'–C–R', in der R' für Wasserstoff oder einen Alkylrest mit 1 bis 4 C-Atomen

$R_1$
|
–O–, –N–, –S– oder –$SO_2$– steht und

m und n für eine ganze Zahl von 1 bis 200, stehen,

das dadurch gekennzeichnet ist, daß ein Gemisch der Phenole der Formel (II) und (III)

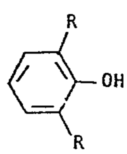

(II)

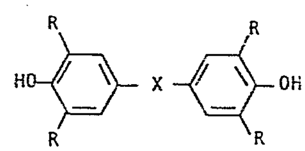

(III)

EP 0 122 412 A1

BAYER AKTIENGESELLSCHAFT 5090 Leverkusen, Bayerwerk

Konzernverwaltung RP
Patentabteilung Eck/by-c

Verfahren zur Herstellung von bifunktionellen Polyphenylenethern

Die Erfindung betrifft ein Verfahren zur Herstellung von bifunktionellen Polyphenylenethern (Polyphenylenoxiden).

Polyphenylenether (bzw. Polyphenylenoxide) sowie mehrere Verfahren zu ihrer Herstellung sind bereits bekannt (z.B. J. Am. Chem. Soc. 81, 6335 (1959), J. Polym. Sci. 58, 581 (1962), US-PS 3 306 879, US-PS 39 14 266, US-PS 39 56 442, US-PS 39 65 069.

So beschreibt beispielsweise die NL-PS 64 13 958 ein Verfahren zur Herstellung von Polyphenylenethern, bei dem monofunktionelle, d.h. eine OH-Gruppe tragende Polyphenylenether die außerdem Halogensubstituenten tragen mit NaOH und KOH zu mehrfunktionellen, d.h. mehrere OH-Gruppen tragenden Polyphenylenethern umgesetzt werden.

Aus der DE-OS 28 22 856 ist ein Verfahren zur Herstellung von Polyphenylenoxiden mit zwei Hydroxylendgruppen bekannt. Bei diesem Verfahren erhalten Polyphenylenoxide, die nur eine Hydroxylgruppe besitzen, durch den Einbau von Chinonen eine weitere Hydroxylgruppe.

Die vorliegende Erfindung betrifft ein Verfahren zur Herstellung von bifunktionellen Polyphenylenoxiden der Formel (I)

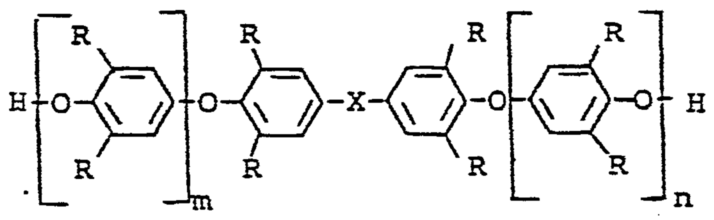

(I),

in welcher

R gleich oder verschieden ist und für Wasserstoff, einen Alkylrest mit 1 bis 4 C-Atomen, vorzugsweise Wasserstoff oder Methyl oder einen Arylrest mit 6 C-Atomen steht,

X für eine Gruppe $R^1-\underset{|}{\overset{|}{C}}-R^1$, in der $R^1$ für Wasserstoff oder einen Alkylrest mit 1 bis 4 C-Atomen, -O-, $-\overset{R^1}{\overset{|}{N}}-$, -S- oder $-SO_2-$ stehen und

m und n für eine ganze Zahl von 1 bis 200, vorzugsweise 5 bis 60 steht,

das dadurch gekennzeichnet ist, daß ein Gemisch der Phenole der Formeln (II) und (III)

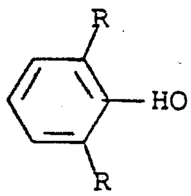 (II), 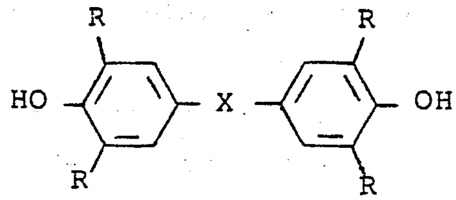 (III),

in welchen

R gleich oder verschieden ist und die bei Formel (I) angegebene Bedeutung hat,

in einem organischen Lösungsmittel, in Gegenwart eines Katalysators bei einer Temperatur von -80 bis 100°C, gegebenenfalls unter erhöhtem Druck, mit Sauerstoff umgesetzt werden.

Als organische Lösungsmittel können aromatische Lösungsmittel wie Benzol, Toluol, Ethylbenzol, Nitrobenzol, halogenierte Lösungsmittel wie Tetrachlorkohlenstoff, Dichlorethan, Trichlorethylen, Tetrachlorethylen, Tetrachlorethan, Trichlorethan, Chlorbenzol und Pyridine eingesetzt werden.

Als Katalysatoren können Metallverbindungen, z.B. Halogenide, Sulfate und Oxide von Elementen der 1. und 7. Nebengruppe des Periodensystems der Elemente nach Mendelejew, (Hofmann Rüdorff, Anorganische Chemie, 19. Auflage, 1963 Seite 97, Vieweg Verlag, Braunschweig), wie CuCl, CuBr, $Cu_2SO_4$, $CuCl_2$, $MnCl_2$, $Ag_2O$ eingesetzt werden. Weiterhin können organische Basen wie Pyridin, Methylpyridin, N,N-Dimethyl-4-aminopyridin,

Poly-4-vinylpyridin, Piperidin, Morpholin, Triethanolamin sowie offenkettige aliphatische Amine wie n-Butylamin, Octylamin, Dibutylamin, N,N-Dimethyl-n-hexylamin, N,N-Dimethyl-n-butylamin, Triethylamin, (N,N'-Di-tert.-butyl) ethylendiamin, 2-Aminoethanthiol, 2-Mercapto-1-ethanol, 2-Mercaptoessigsäure, 1,2-Dimercapto-4-methylbenzol, Dinatrium-1,2-dicyanoethylendithiolat, Dimercaptomaleinsäuremonoamid, Schiffsche Basen und Hydrazone, z.B. Hydrazone des Benzoins, polymere Komplexe, die Bis-(ethylen-1,2-dithiolate/Cu II und Cu-II-Phthalocyanin-Strukturen) enthalten, als Cokatalysater verwendet werden.

Die erfindungsgemäße Umsetzung wird bei einer Temperatur von -80°C bis 100°C, bevorzugt 0 - 40°C und einem Druck von 0 bis 15 bar, bevorzugt 0 bis 5 bar durchgeführt.

Pro Mol Phenol der Formel (III) werden 2 bis 400 Mol Phenol der Formel (II) eingesetzt.

Das erfindungsgemäße Verfahren kann wie folgt durchgeführt werden:

Der Katalysator und der Cokatalysator werden im organischen Lösungsmittel vorgelegt und mit Sauerstoff oxidiert. Anschließend wird das Monomergemisch der Phenole der Formel (II) und (III) im organischen Lösungsmittel gelöst zugegeben und mit Sauerstoff oxidiert.

Dann wird diese Mischung einige Minuten bis mehrere Tage bei Reaktionstemperatur belassen. Dann wird, beispielsweise durch Ausfällen mit einem Alkohol (z.B. Methanol), das bifunktionelle Polyphenylenoxid isoliert. Die Analyse des Reaktionsproduktes kann auf übliche Weise erfolgen (z.B. osmometrische Molgewichtbestimmung, Bestimmung der phenolischen OH-Zahl z.B. durch Titration).

Durch das erfindungsgemäße Verfahren können sowohl symmetrische und unsymmetrische bifunktionelle Polyphenylenether hergestellt werden. Im Sinne der Erfindung symmetrische Produkte werden erhalten, wenn Phenole der Formel (II) und (III) eingesetzt werden, die gleiche Substituenten tragen.

Durch das erfindungsgemäße Verfahren können Polymerblöcke mit zwei funktionellen Endgruppen hergestellt werden, die eine hohe Glasübergangstemperatur besitzen. Diese Blöcke können beispielsweise mit Säurechloriden wie Phosgen, Terephthaloylchlorid, mit Diisocyanaten, mit Carbonaten und anderen Polymerblöcken zu hochmolekularen Verbindungen umgesetzt werden. Materialien, die aus diesen hochmolekularen Verbindungen hergestellt werden, zeichnen sich durch ihre gute Wärmeformbeständigkeit aus.

## Beispiele

### Beispiel 1

In die Reaktionsapparatur, bestehend aus einem 500 ml Dreihalskolben, KPG-Rührer, Gaseinleitungsrohr und Gasabgangsstück, werden 0,184 g CuCl (1,86 mmol), 0,332 g 4-Dimethylaminopyridin (2,72 mmol) und 50 ml Chloroform gegeben. In diese Mischung wird 15 Minuten lang unter Rühren $O_2$ mit einer Flußrate von 15 l/h eingeleitet. Zu dieser aktivierten Kupfer-Amin-Katalysatorlösung wird bei Raumtemperatur eine Lösung von 7 g 2,6-Dimethylphenol (57,3 mmol) und 4,1 g 2,2-Bis-(3,5-dimethyl-4-hydroxy-phenyl)-propan (14,4 mmol) in 90 ml Chloroform zugegeben.

Die Sauerstoffzufuhr wird für 2 h bei Raumtemperatur fortgeführt und dann beendet. Die Aufarbeitung der Reaktionslösung geschieht durch jeweils zweimaliges Ausschütteln mit je 20 ml einer 20 %igen wäßrigen EDTA[a)] Trinatriumsalzlösung, einer 15 %igen wäßrigen HCl-Lösung, einer 10 %igen wäßrigen $NaHCO_3$-Lösung und nachfolgend mit destilliertem Wasser. Nach dem Trocknen der so behandelten Reaktionslösung über $MgSO_4$ wird das Lösungsmittel bei 40°C im Wasserstrahlpumpenvakuum und unreagiertes 2,6-Dimethylphenol bei 70°C und 0,1 Torr im Ölpumpenvakuum weitgehend abdestilliert. Man erhält 9,6 g Oligomere, (87 % der Theorie, Molekulargewichtsmittel $M_n$ = 887, titrierte Funktionalität an phenolischen Hydroxylgruppen von 1,89).

a) EDTA-Ethylendiamintetraacetat

Beispiel 2

In ein 1 l Reaktionsgefäß werden 0,8 g CuCl und 1,7 g 4-Dimethylaminopyridin in 60 ml Chloroform gegeben. Unter Rühren werden 20 Minuten lang Sauerstoff bei einem Fluß von 15 l/h eingeleitet. Eine Lösung von 20 g 2,2-Bis-(3,5-dimethyl-4-hydroxyphenyl)-propan und 35 g 2,6-Dimethylphenol in 270 ml $CHCl_3$ wird dazugegeben. Die Sauerstoffzufuhr wird bei Raumtemperatur für weitere 3 h bei konstantem Fluß belassen. Die Reaktionslösung wird jeweils zweimal mit 40 ml-Portionen einer 20 %igen wäßrigen EDTA-trinatriumsalzlösung, einer 15 %igen wäßrigen HCl-Lösung, einer 10 %igen wäßrigen $NaHCO_3$-Lösung und destilliertem Wasser gewaschen.

Nach Trocknen und Abdestillieren des Lösungsmittels und unverbrauchtem Monomerem werden 49,7 g Polyphenylenoxid-Oligomere erhalten (90,3 % der Theorie, Molekulargewichtsmittel $M_n$ = 395, titrierte Funktionalität an phenolischen Hydroxylgruppen 1,63).

Beispiel 3

0,4 g CuCl und 1 g 4-Dimethylaminopyridin werden mit 60 ml $CHCl_3$ in ein 1 l Reaktionsgefäß gegeben. Durch diese Katalysatorlösung wird unter Rühren ein Sauerstoffstrom mit einer Flußrate von 15 l/h durchgeleitet. Nach 20 Minuten wird bei Raumtemperatur eine Lösung von 10,25 g 2,2-Bis-(3,5-dimethyl-4-hydroxyphenyl)-methan (40,0 mmol) und 30,1 g 2,6-Dimethylphenol (24,6 mmol) in 320 ml Chloroform dazugegeben. Die $O_2$-Zufuhr wird nach 3 h

beendet und die Reaktionslösung analog zu Beispiel 2 aufgearbeitet. Nach Abdestillieren von Lösungsmittel und unverbrauchtem 2,6-Dimethylphenol werden 36,1 g Oligomere erhalten (91,1 % der Theorie, dampfdruckosmometrisch bestimmtes Molekulargewichtsmittel $M_n$ 518, titrierte Funktionalität der phenolischen OH-Gruppen 1,52).

Patentansprüche

1. Verfahren zur Herstellung von bifunktionellen Polyphenylenoxiden der Formel (I)

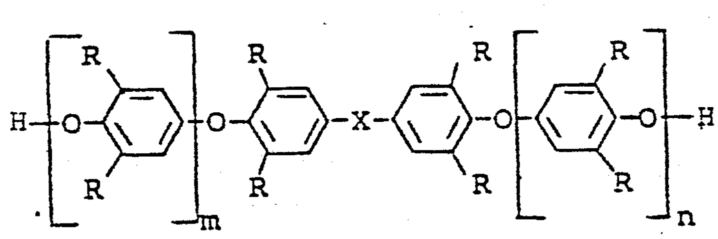

(I),

in welcher

R gleich oder verschieden ist und für Wasserstoff, einen Alkylrest mit 1 bis 4 C-Atomen, oder einen Arylrest mit 6 C-Atomen steht,

X für eine Gruppe $R^1-\overset{|}{\underset{|}{C}}-R^1$, in der $R^1$ für Wasserstoff oder einen Alkylrest mit 1 bis 4 C-Atomen

-O-, $-\overset{R_1}{\overset{|}{N}}-$, -S- oder $-SO_2-$ steht und

m und n für eine ganze Zahl von 1 bis 200, stehen,

das dadurch gekennzeichnet ist, daß ein Gemisch der Phenole der Formel (II) und (III)

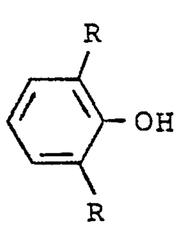

(II)

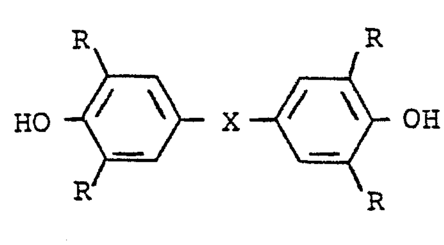

(III)

in welchen

R gleich oder verschieden ist und die bei Formel (I) angegebene Bedeutung hat,

in einem organischen Lösungsmittel, in Gegenwart eines Katalysators bei einer Temperatur von -80 bis 100°C, gegebenenfalls unter erhöhtem Druck mit Sauerstoff umgesetzt werden.

2. Verwendung von Polyphenylenoxiden gemäß Anspruch 1 zur Modifizierung von Kunststoffen.

Europäisches Patentamt

# EUROPÄISCHER RECHERCHENBERICHT

0122412
Nummer der Anmeldung

EP 84 10 2010

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | CHEMICAL ABSTRACTS, Band 82, Nr. 4, 27. Januar 1975, Seite 65, Nr. 17960d, Columbus, Ohio, USA; & JP - A - 74 59154 (ASAHI DOW LTD.) 08.06.1974 * Zusammenfassung * --- | 1,2 | C 08 G 65/44<br>C 07 C 43/295<br>C 07 C 41/01 |
| X | CHEMICAL ABSTRACTS, Band 82, Nr. 4, 27. Januar 1975, Seite 52, Nr. 17798g, Columbus, Ohio, USA; & JP - A - 74 69 797 (ASAHI DOW LTD.) 05.07.1974 * Zusammenfassung * ----- | 1 | |
| | | | **RECHERCHIERTE SACHGEBIETE (Int. Cl. 3)** |
| | | | C 07 C 41/00<br>C 07 C 43/00<br>C 08 G 65/00 |

Der vorliegende Recherchenbericht wurde für alle Patentansprüche erstellt.

| Recherchenort | Abschlußdatum der Recherche | Prüfer |
|---|---|---|
| DEN HAAG | 15-06-1984 | WRIGHT M.W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82